# EUROPEAN PATENT APPLICATION

(11) **EP 2 573 163 A1**
(43) Date of publication of application: **27.03.2013**
(21) Application number: 12185626.4
(22) Date of filing: 24.09.2012
(51) Int. Cl.: C12M 1/00

(54) **Culture apparatus having heaters**

(30) Priority: 22.09.2011 JP 2011207840
(71) Applicant: Panasonic Healthcare Co., Ltd., Ehime 791-0395 (JP)
(72) Inventor: Yamasaki, Naoyuki, Chuo-ku, Osaka 540-6207 (JP); Kikuchi, Yasuhiro, Chuo-ku, Osaka 540-6207 (JP); Busujima, Hiroki, Chuo-ku, Osaka 540-6207 (JP)
(74) Representative: Grünecker, Kinkeldey, Stockmair & Schwanhäusser

(57) **Abstract**

Provided is a culture apparatus capable of regaining a prescribed temperature within a short period of time even if a temperature inside a culture vessel is decreased below the prescribed temperature and generating no temperature unevenness inside the culture vessel. The problem can be solved by a culture apparatus including : a heat insulating box main body having an opening on a front side thereof; a heat insulating door attached to the heat insulating box main body in an openable and closable manner; a transparent inner door that seals the opening in an openable and closable manner; a culture vessel for culturing a sample such as cells or microorganisms, the culture vessel being surrounded by the inner door and the heat insulating box main body; a duct and a circulation blower that are used to cause forced convection of a gas such as air in the culture vessel; and a humidifying pan disposed on a bottom portion of the culture vessel within the duct, the humidifying pan being configured to store humidifying water used to control humidity inside the culture vessel, wherein the heat insulating box main body includes an outer box made of a metal, an inner box made of a metal, a heat insulator disposed between the outer box and the inner box and on an inner side of the outer box, an air layer disposed on an inner side of the heat insulator, and heaters disposed on the outer side of the inner box and on the inner side of the heat insulating door, for heating the inside of the culture vessel. The culture apparatus is configured such that a rapid heater is provided on the downstream side of the circulation blower inside the duct, and the rapid heater is activated if a temperature in the culture vessel is decreased below a prescribed temperature, thereby rapidly regaining the prescribed temperature.

## Description

### BACKGROUND OF THE INVENTION

### 1. Field of the Invention

The present invention relates to a culture apparatus having heaters.

### 2. Description of the Related Art

There is a culture apparatus for culturing cultures such as cells or microorganisms in a culture vessel. This culture apparatus is provided with a heater for heating the inside of the culture vessel where a humidifying pan is disposed. This culture apparatus is configured, for example, to control the heater so as to maintain the inside of the culture vessel at a prescribed temperature (for example, at 37°C) and also to maintain the inside thereof at a prescribed humidity (for example, 95%RH) corresponding to this prescribed temperature.

There has been also disclosed, for example, a culture apparatus including: a bottom heater for heating water in a humidifying pan; a heater for heating the inside of a culture vessel excluding the humidifying pan; and a heater attached to a heat insulating door which is attached to a heat insulating box main body in an openable and closable manner. These three heaters are individually controlled to maintain the temperature of water in the humidifying pan at a temperature lower than that in the culture vessel so as to return supersaturated water in the culture vessel to the humidifying pan, thereby suppressing dew condensation (for example, see Patent Literature 1). This culture apparatus includes a temperature sensor for detecting a temperature inside the culture vessel and a temperature sensor for detecting an ambient temperature. Based on the detection results obtained by these two temperature sensors, the above-described three kinds of heaters are individually controlled.

### [Citation List]

### [Patent Literature]

Patent Literature 1: Japanese Patent Application Laid-open No. 5-227942

However, if a large number of culture dishes containing cultures such as cells or microorganisms therein, or the like, are put in the culture vessel in order to culture the cultures, a temperature inside the culture vessel is thereby decreased below a prescribed temperature (for example, 37°C). Thus, there is a problem that it takes a long time to return to the above-described prescribed temperature only by means of the existing heaters. If it takes a long time to return to the prescribed temperature, dew condensation may occur in the vicinity of the cultures, for example, and bacteria may be generated in the dew condensation water. Thus, there occurs a problem that the cultures are adversely affected.

Thus, if an internal heater is disposed inside the culture vessel, there occurs a problem that temperature unevenness may be generated in the culture vessel, thereby adversely affecting the cultures in the culture vessel.

### SUMMARY OF THE INVENTION

An object of the present invention is to provide a culture apparatus capable of solving the conventional problems and capable of regaining a prescribed temperature within a short period of time even if a temperature inside a culture vessel is decreased below the prescribed temperature, and also generating no temperature unevenness inside the culture vessel.

As a result of the intensive study for solving the above-described problem, the inventor has found that the above-described problem can be solved by providing, in a duct having a circulation blower for obtaining forced convection of a gas such as air in a culture vessel, a rapid heater on the downstream side of the circulation blower and performing the control thereof, thereby arriving at the present invention.

The invention according to claim 1 for solving the above-described problem is a culture apparatus including: a heat insulating box main body having an opening on a front side thereof; a heat insulating door attached to the heat insulating box main body in an openable and closable manner; a transparent inner door that seals the opening in an openable and closable manner; a culture vessel for culturing a sample such as cells or microorganisms, the culture vessel being surrounded by the inner door and the heat insulating box main body; a duct and a circulation blower that are used to cause forced convection of a gas such as air in the culture vessel; and a humidifying pan disposed on a bottom portion of the culture vessel within the duct, the humidifying pan being configured to store humidifying water used to control humidity inside the culture vessel, wherein the heat insulating box main body includes an outer box made of a metal, an inner box made of a metal, a heat insulator disposed between the outer box and the inner box and on an inner side of the outer box, an air layer disposed on an inner side of the heat insulator, and heaters disposed on the outer side of the inner box and on the inner side of the heat insulating door, for heating the inside of the culture vessel, and wherein a rapid heater is provided on the downstream side of the circulation blower inside the duct, and the rapid heater is activated if a temperature in the culture vessel is decreased below a prescribed temperature, thereby rapidly regaining the prescribed temperature.

In a second aspect of the invention, the culture apparatus of the first aspect is configured such that the rapid heater is activated while being controlled so that a temperature of the rapid heater itself is maintained at the prescribed temperature in the culture vessel after the temperature in the culture vessel is reached to the prescribed temperature.

### Advantageous Effects of Invention

The culture apparatus according to the first aspect of the invention includes: a heat insulating box main body having an opening on a front side thereof; a heat insulating door attached to the heat insulating box main body in an openable and closable manner; a transparent inner door that seals the opening in an openable and closable manner; a culture vessel for culturing a sample such as cells or microorganisms, the culture vessel being surrounded by the inner door and the heat insulating box main body; a duct and a circulation blower that are used to cause forced convection of a gas such as air in the culture vessel; and a humidifying pan disposed on a bottom portion of the culture vessel within the duct, the humidifying pan being configured to store humidifying water used to control humidity inside the culture vessel, wherein the heat insulating box main body includes an outer box made of a metal, an inner box made of a metal, a heat insulator disposed between the outer box and the inner box and on an inner side of the outer box, an air layer disposed on an inner side of the heat insulator, and heaters disposed on the outer side of the inner box and on the inner side of the heat insulating door, for heating the inside of the culture vessel. This culture apparatus is configured such that a rapid heater is provided on the downstream side of the circulation blower inside the duct, and the rapid heater is activated if a temperature in the culture vessel is decreased below a prescribed temperature, thereby rapidly regaining the prescribed temperature. By providing the rapid heater on the downstream side of the circulation blower in the duct and by activating the rapid heater if a temperature in the culture vessel is decreased below the prescribed temperature, there can be obtained prominent effects of being able to regain the prescribed temperature within a short period of time and generating no temperature unevenness in the culture vessel.

In the second aspect of the invention, the culture apparatus according to the first aspect is configured such that the rapid heater is activated while being controlled so that a temperature of the rapid heater itself is maintained at the prescribed temperature in the culture vessel after the temperature in the culture vessel is reached to the prescribed temperature. If the rapid heater is turned OFF after the temperature in the culture vessel has reached the prescribed temperature, the temperature of the rapid heater itself may possibly be decreased below the prescribed temperature in the culture vessel, thereby resulting in the dew condensation in the rapid heater. However, if the temperature control is made so as to maintain the temperature of the rapid heater itself at the prescribed temperature in the culture vessel by passing a small amount of electric current therethrough, for example, there can be obtained a further prominent effect of preventing the dew condensation.

### BRIEF DESCRIPTION OF THE DRAWINGS

FIG. 1 is a perspective view illustrating a culture apparatus of the present invention provided with heaters when a heat insulating door of the culture apparatus is opened; and
FIG. 2 is a cross-sectional view for mainly showing a culture vessel and a duct of the culture apparatus having heaters according to the present invention and for explaining air circulation therein in a case where the culture vessel is viewed from the right side.

### DETAILED DESCRIPTION OF THE PREFERRED EMBODIMENT

An embodiment of the present invention will now be described below in detail with reference to the drawings.

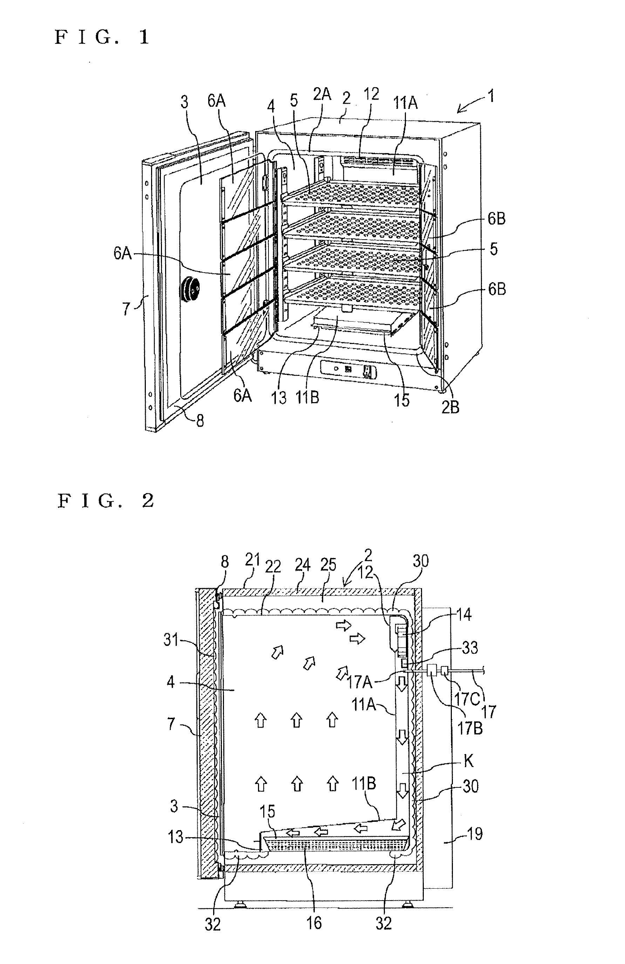

A culture apparatus 1 according to the embodiment of the present invention includes doors that open from the right (more specifically, an outer door and an inner door) and small double doors as shown in FIGs. 1 and 2. A culture vessel 4 is formed as a space defined by a heat insulating box main body 2 having an opening 2A on its front side and a transparent door 3 used as an inner door that can seal the opening 2A in an openable and closable manner. The left side of the transparent door 3 is hinged to the heat insulating box main body 2 in an openable and closable manner, and the transparent door 3 can hermetically seal the opening 2A through a gasket 2B disposed on an opening portion of the culture vessel 4. The sealing member (gasket) 2B for sealing the transparent inner door 3 and the heat insulating box main body 2 is disposed on the opening of the culture vessel 4.

The inside of the culture vessel 4 is vertically partitioned by a plurality of shelves 5 (into 5 sections by 4 shelves in this embodiment). When the culture apparatus 1 is, for example, a CO₂ incubator, the concentration of CO₂ is often set to and maintained at about 5%, and CO₂ gas is supplied to the inside of the culture vessel 4 to control the concentration of CO₂ after the doors are hermetically sealed. Therefore, a plurality of pairs of small double doors 6A and 6B for the plurality of partitioned sections (5 pairs in this embodiment) are provided on the inner side of the inner door 3 so that outside air is prevented from entering the entire culture vessel 4 partitioned into the partitioned sections even when the inner door 3 is opened. Reference numeral 7 denotes a heat insulating door serving as an outer door hinged to the heat insulating box main body 2 in an openable and closable manner and used to prevent heat from entering from the opening 2A of the culture vessel 4, and a gasket 8 with a magnet is provided on the rear circumference of the heat insulating door 7.

In the culture vessel 4, a duct 11 composed of a rear-side duct 11A and a bottom duct 11B is disposed on the rear side and the bottom of the culture vessel 4 so as to form a space for a gas passage K for air etc. A gas such as air etc. in the culture vessel 4 is drawn from an inlet port 12 formed in the upper part of the rear-side duct 11A and is blown out into the culture vessel 4 from an outlet port 13 disposed over the front and side surfaces of the bottom duct 11B, so that the gas is forcibly circulated. Inside the duct 11 (in the upper part thereof in this embodiment), a circulation blower 14 is provided for the purpose of the forced circulation of a gas such as air. The blower 14 is composed of a fan, a motor, and a shaft. The motor is disposed in a machine room 19 (described later) on the outer rear side of the culture vessel 4, and the shaft extends from the motor in the machine room 19, passes through the rear surface of the heat insulating box main body 2, reaches the gas passage K for air etc., and is connected to the fan.

A heater 30 for heating the inside of the culture vessel 4 is provided on the outer side of an inner box 22 made of a metal, and a heater 31 for heating the inside of the culture vessel 4 is also provided on the inner side of the heat insulating door 7. A humidifying pan 15 for storing humidifying water 16 (i.e., water used for humidification) is disposed on the bottom of the culture vessel 4 within the duct 11. The humidifying pan 15 is heated by a heater 32 disposed on the outer bottom side of an inner box 22 made of a metal (for example, stainless steel), and the water thereby evaporates. By disposing the humidifying pan 15 on the bottom of the culture vessel 4 within the duct 11, a humidified gas in the gas passage K for air etc. that is composed of the circulation blower 14 and the duct 11 can be more efficiently blown out into the culture vessel 4.

Formed on the rear side of an outer box 21 of the heat insulating box main body 2 is the mechanical chamber 19 for arranging therein the motor serving as driving means for the circulation blower 14, gas supply means 17 for supplying a gas such as a CO₂ gas or air into the culture vessel 4, and electric components (not shown) such as a control board.

The gas supply means 17 is composed of a gas supply tube 17A, an on-off valve 17B, a filter 17C, and the like. The tip portion of the gas supply tube 17A is positioned in the gas passageway K.

The CO₂ gas supplied from the gas supply tube 17A can be injected into the culture vessel 4 in order to control the gas concentration inside the culture vessel 4.

The heat insulating box main body 2 includes: the outer box 21 made of a metal; the inner box 22 made of a stainless steel; a heat insulator 24 disposed between the outer box 21 and the inner box 22 and positioned on the inner side of the outer box 21; and an air layer (what is called an air jacket) 25 disposed on the inner side of the heat insulator 24. Heaters (not shown) for heating the culture vessel are disposed on both the sides, top side, and rear side of the inner box 22 forming the culture vessel 4.

The culture apparatus 1 of the present invention is configured so that a rapid heater 33 is provided on the downstream side of the circulation blower 14 inside the duct 11 as shown in FIG. 2. If the temperature inside the culture vessel 4 is detected by a temperature sensor (not shown) and is decreased below a prescribed temperature, such information is transmitted to a controller (not shown). Then, the rapid heater 33 is activated by a signal from the controller so as to start heating, thereby rapidly regaining the prescribed temperature.

The rapid heater 33 is provided on the downstream side of the circulation blower 14 inside the duct 11. In a case where a temperature inside the culture vessel 4 is decreased below the prescribed temperature, the rapid heater 33 is activated and the forced circulation of air is conducted so that a gas such as air inside the culture vessel 4 is sucked in by the circulation blower 14 from the inlet 12 formed at the upper part of the rear-side duct 11A and the gas heated by the rapid heater 33 is blown out, through the duct 11 including the rear-side duct 11A and the bottom duct 11B, into the culture vessel 4 from the outlet 13 disposed over the front and side surfaces of the bottom duct 11B. As a result, the temperature inside the culture vessel 4 can regain the prescribed temperature within a short period of time and no temperature unevenness is generated in the culture vessel 4.

If the rapid heater 33 is turned OFF after the temperature in the culture vessel 4 has reached the prescribed temperature, the temperature of the rapid heater 33 itself may possibly be decreased below the prescribed temperature in the culture vessel 4, thereby resulting in the dew condensation in the rapid heater 33. If the dew condensation occurs, bacteria or the like may possibly grow, thereby interfering with the cultivation of the culture. To cope with this, the dew condensation can be prevented if the temperature control is made, after the temperature in the culture vessel 4 is reached to the prescribed temperature, so as to maintain the temperature of the rapid heater 33 itself at the prescribed temperature in the culture vessel 4 by passing a small amount of electric current therethrough, for example.

An electric capacitance, a shape, a size, the number, and the like of the rapid heater used in the present invention vary depending upon a capacitance, a shape, a size, a type of culture, and the like of the culture apparatus.

The above described embodiment of the invention is for illustrative purposes only and is not intended to limit the claims and to reduce the scope of the invention. The configuration of each component is not limited to that in the above embodiment and can be modified variously within the technical scope described in the claims.

### Industrial Applicability

According to the present invention, there is provided a culture apparatus including: a heat insulating box main body having an opening on a front side thereof; a heat insulating door attached to the heat insulating box main body in an openable and closable manner; a transparent inner door that seals the opening in an openable and closable manner; a culture vessel for culturing a sample such as cells or microorganisms, the culture vessel being surrounded by the inner door and the heat insulating box main body; a duct and a circulation blower that are used to cause forced convection of a gas such as air in the culture vessel; and a humidifying pan disposed on a bottom portion of the culture vessel within the duct, the humidifying pan being configured to store humidifying water used to control humidity inside the culture vessel, wherein the heat insulating box main body includes an outer box made of a metal, an inner box made of a metal, a heat insulator disposed between the outer box and the inner box and on an inner side of the outer box, an air layer disposed on an inner side of the heat insulator, and heaters disposed on the outer side of the inner box and on the inner side of the heat insulating door, for heating the inside of the culture vessel. In this culture apparatus, a rapid heater is provided on the downstream side of the circulation blower inside the duct, and the rapid heater is activated if a temperature in the culture vessel is decreased below a prescribed temperature, thereby rapidly regaining the prescribed temperature. By providing the rapid heater on the downstream side of the circulation blower in the duct and by activating the rapid heater if a temperature in the culture vessel is decreased below the prescribed temperature, there can be obtained prominent effects of being able to regain the prescribed temperature within a short period of time and generating no temperature unevenness in the culture vessel. Therefore, the industrial utility value thereof is tremendous.

## Claims

1. A culture apparatus comprising:
a heat insulating box main body having an opening on a front side thereof;
a heat insulating door attached to the heat insulating box main body in an openable and closable manner;
a transparent inner door that seals the opening in an openable and closable manner;
a culture vessel for culturing a sample such as cells or microorganisms, the culture vessel being surrounded by the inner door and the heat insulating box main body;
a duct and a circulation blower that are used to cause forced convection of a gas such as air in the culture vessel; and
a humidifying pan disposed on a bottom portion of the culture vessel within the duct, the humidifying pan being configured to store humidifying water used to control humidity inside the culture vessel, wherein
the heat insulating box main body includes an outer box made of a metal, an inner box made of a metal, a heat insulator disposed between the outer box and the inner box and on an inner side of the outer box, an air layer disposed on an inner side of the heat insulator, and heaters disposed on the outer side of the inner box and on the inner side of the heat insulating door, for heating the inside of the culture vessel, and wherein
a rapid heater is provided on the downstream side of the circulation blower inside the duct, and the rapid heater is activated if a temperature in the culture vessel is decreased below a prescribed temperature, thereby rapidly regaining the prescribed temperature.

2. The culture apparatus according to claim 1, wherein the rapid heater is activated while being controlled so that a temperature of the rapid heater itself is maintained at the prescribed temperature in the culture vessel after the temperature in the culture vessel is reached to the prescribed temperature.
